# EUROPEAN PATENT SPECIFICATION

(11) **EP 3 813 801 B1**
(45) Date of publication and mention of the grant of the patent: **29.07.2026**
(21) Application number: 19855155.8
(22) Date of filing: 24.05.2019
(51) Int. Cl.: A61K 9/00, A61K 9/22, A61K 9/24, A61K 31/155, A61K 31/4985, A61P 3/10

(54) **EXTENDED RELEASE OSMOTIC TABLET DOSAGE FORM COMPRISING METFORMIN AND SITAGLIPTIN**
OSMOTISCHE TABLETTENDARREICHUNGSFORM MIT VERLÄNGERTER FREISETZUNG MIT METFORMIN UND SITAGLIPTIN
FORME POSOLOGIQUE DE COMPRIMÉ OSMOTIQUE À LIBÉRATION PROLONGÉE COMPRENANT DE LA METFORMINE ET DE LA SITAGLIPTINE

(30) Priority: 25.05.2018 TR 201807484
(43) Date of publication of application: 05.05.2021
(73) Proprietor: Sanovel Ilac Sanayi ve Ticaret A.S., Sisli - Istanbul (TR)
(72) Inventor: TURKYILMAZ, Ali, 34460 Istanbul (TR); ATAMAN, Seval, 34460 Istanbul (TR); OZTURK, Erkin, 34460 Istanbul (TR); TASKIN, Abdullah, 34460 Istanbul (TR)
(74) Representative: Genç Ilhan, Oznur
(86) International application number: PCT/TR2019/050378
(87) International publication number: WO 2020/046243

(56) References cited:
- WO-A1-2016/016770
- CN-A- 107 669 683
- KR-A- 20160 111 237
- US-A1- 2007 172 525
- US-A1- 2015 366 863
- RAJESH A. KERALIYA ET AL: "Osmotic Drug Delivery System as a Part of Modified Release Dosage Form", ISRN PHARMACEUTICS, vol. 2012, 1 January 2012 (2012-01-01), pages 1 - 9, XP055545092, DOI: 10.5402/2012/528079
- PATEL APURV ET AL: "Design, Characterization, and Optimization of Controlled Drug Delivery System Containing Antibiotic Drug/s", vol. 2016, 16 August 2016 (2016-08-16), pages 1 - 15, XP055893838, ISSN: 2090-3014, Retrieved from the Internet <URL:https://www.ncbi.nlm.nih.gov/pmc/articles/PMC5004061/pdf/JDD2016-9024173.pdf> DOI: 10.1155/2016/9024173
- ANONYMOUS: "Assessment Report for Janumet (EMEA/H/C/000861)", EMEA, 1 January 2008 (2008-01-01), pages 1 - 43, XP055591802
- LIN SHAN-YANG ET AL: "Influence of excipients, drugs, and osmotic agent in the inner core on the time-controlled disintegration of compression-coated ethylcellulose tablets", JOURNAL OF PHARMACEUTICAL SCIENCES, vol. 91, no. 9, 1 September 2002 (2002-09-01), US, pages 2040 - 2046, XP055893843, ISSN: 0022-3549, DOI: 10.1002/jps.10197
- PUJARI PRAKASH S ET AL: "Formulation Development and Evaluation of Bilayer Floating Tablet of Antidiabetic Drugs", 1 January 2016 (2016-01-01), pages 34 - 54, XP055894064, Retrieved from the Internet <URL:https://www.scholarsresearchlibrary.com/articles/formulation-development-and-evaluation-of-bilayer-floating-tablet-of-antidiabetic-drugs.pdf> [retrieved on 20220221]
- CHINMAYA KESHARI SAHOO ET AL: "A review on controlled porosity osmotic pump tablets and its evaluation", CAIRO UNIVERSITY. FACULTY OF PHARMACY. BULLETIN, vol. 53, no. 2, 1 December 2015 (2015-12-01), Egypt (Arab Republic of Egypt), pages 195 - 205, XP055589302, ISSN: 1110-0931, DOI: 10.1016/j.bfopcu.2015.10.004
- PRATHIMA SRINIVAS M ET AL: "Formulation and evaluation of sitagliptin phosphate and metformin hydrochloride trilayered tablets", INTERNATIONAL JOURNAL OF DRUG DELIVERY, ADVANCED RESEARCH JOURNALS, US, vol. 5, no. 1, 1 January 2013 (2013-01-01), pages 15 - 27, XP009179090, ISSN: 0975-0215

## Description

### Field of the invention

The present invention relates to an extended release osmotic tablet dosage form comprising metformin and sitagliptin. Further, the present invention provides a method for the preparation of the said composition.

### Background of the invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight. Type 2 diabetes may sometimes be controlled with a combination of diet, weight management, and exercise. However, the treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether, and chloroform. Oral doses of metformin are generally recommended in the range of 500 to 2500 mg a day and a single dose may vary from 250 to 1000 mg. It is used singly or in combination with sulfonylureas, alpha-glucosidase inhibitors, or insulin.

The chemical name of metformin is 1,1-dimethyl biguanide, has the following chemical structure of Formula I.

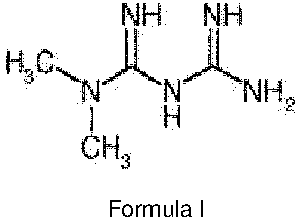

Although metformin is effective at lowering blood glucose levels, its use is associated with gastrointestinal (GI) adverse effects, particularly diarrhea and nausea. These adverse effects may limit the tolerated dose of metformin and cause patients to discontinue the therapy.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (3R)-3-amino-1-[3-(trifluoromethyl)-6,8-dihydro-5H-[1,2,4]triazolo[4,3-a]pyrazin-7-yl]-4-(2,4,5-trifluorophenyl) butan-1-one or (2R)-4-oxo-4- [3-(trifluoromethyl)-5,6-dihydro[1,2,4]triazolo[4,3-a]pyrazin-7(8H)-yl]-1-(2,4,5trifluorophenyl) butan-2-amine) and its chemical structure is shown in the Formula II.

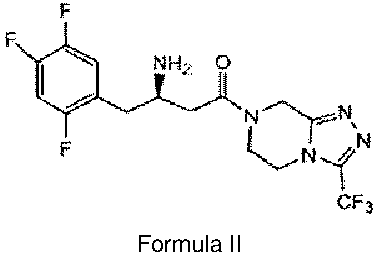

Sitagliptin is disclosed in the U.S. Patent No. 6699871. A crystal phosphate monohydrate form of sitagliptin is disclosed in the patent WO 2005003135.

DPP-4 inhibitors, especially sitagliptin have a novel mechanism of action and many studies showing their efficacy and safety in medication are available.

Sitagliptin increases plasma GLP-1 concentration and elevates cellular cAMP levels in pancreatic beta-cells leading to potentiate insulin secretion, whereas metformin improves glucose tolerance in patients with Type 2 diabetes, lowering both basal and postprandial plasma glucose. Its pharmacologic mechanisms of action are different from other classes of oral antihyperglycemic agents in that metformin decreases hepatic glucose production, decreases intestinal absorption of glucose and improves insulin sensitivity by increasing peripheral glucose uptake and utilization. Therefore, they both help to reduce blood glucose levels in different pathways in type 2 diabetes patients.

Extended-release formulations of metformin have advantages over immediate release in terms of affording more uniform maintenance of blood plasma active drug concentrations and providing better patient compliance by reducing the frequency of administration required.

Extended-release formulations comprising metformin are disclosed in several other U.S. Patent No. 6,635,280 and U.S. Patent No. 6,866,866 and U.S. Patent No. 6,660,300.

A tablet formulation for sitagliptin and extended-release metformin combination is commercially available under the trade name Janumet XR^{®}.

CN 107 669 683 A discloses a composition comprisinf sitagliptin and metformin. According to the teaching of CN 107 669 683 A, a sitagliptin layer cover a core containing metformin. However, sodium chloride and calcium chloride are mentioned within CN 107 669 683 A. Par. [0090] of CN 107 669 683 A discloses sodium chloride while describing the dissolution method for sitagliptin and metformin. Calcium chloride is disclosed in par. [0007] of CN 107 669 683 A; which is mentioned to be in the DPP-IV layer.

Pujari Prakash S et. al. is about bilayer floating tablets of antidiabetic drugs, has no explicit disclosure about osmotic agents.

US 2015/366863 A1 describes pharmaceutical compositions comprising sitagliptin and metformin wherein the composition devoid of glidant and/or surface-active agent. Osmotic agents are not referred literally within US 2015/366863 A1. Only sorbitol and xylitol are cited within the teaching of US 2015/366863 A1.

According to the par. [0063] of US 2015/366863 A1, sorbitol is cited among suitable alternatives for filler and filler-binder. No further teaching is given for sorbitol and US 2015/366863 A1 does not disclose sorbitol to be used in the core of the dosage form together with metformin. Xylitol is only cited as a filler-binder alternative in par. [0063] of US 2015/366863 A1; however, US 2015/366863 A1 is silent about the use of xylitol in the core zone of the dosage form together with metformin.

PCT publication WO 2009111200 discloses the tablet comprising the combinations of an extended-release form of metformin, or a pharmaceutically acceptable salt thereof, coated with an immediate-release form of sitagliptin, or a pharmaceutically acceptable salt thereof. WO 2009099734 discloses pharmaceutical compositions providing an extended release of metformin and an immediate release of sitagliptin. The tablet core is comprised of metformin and an extended release polymer (HPMC). The tablet core is then coated with immediate release polymer comprising sitagliptin.

There is also still a need for a tablet form comprising metformin and sitagliptin having the desired dissolution profile and stability.

In the present invention, the dosage form of pharmaceutical combination is the extended release osmotic tablet to overcome the problem of incompatibility and dissolution rate; wherein dosage form comprises a) a core having metformin and the osmotic agent; b) a semipermeable membrane coating surrounding the core, and c) an immediate release drug coating having sitagliptin surrounding the semipermeable membrane coating and wherein the amount of metformin is between 40.0% and 90.0% by weight of the dosage form, the amount of sitagliptin is between 2.0% and 15.0% by weight of the dosage form and wherein the amount of the osmotic agent in the core is between 1.0% and 10.0% by weight and wherein the osmotic agents comprise sodium chloride, magnesium sulfate, magnesium chloride, calcium chloride, lithium chloride, potassium chloride, sorbitol, xylitol, glucose, fructose or mixtures thereof. It has been surprisingly observed that an unexpected therapeutic benefit and especially a synergistic therapeutic benefit can be obtained in the treatment of type-2 diabetes by said extended release osmotic tablet dosage form, while minimizing the problem of dissolution rate and stability. Also, the tablet provides the desired dissolution profile without extended release polymers.

### Detailed description of the Invention

The main object of the present invention is to avoid incompatibility problems between metformin and sitagliptin using the osmotic tablet and to provide the desired stability and the desired dissolution rate in the osmotic tablet.

It is also a further object of the present invention to provide a dosage form comprising delivery of a first active drug as an extended release formulation for metformin in combination with delivery of a second active drug as immediate release comprising sitagliptin wherein the tablet does not comprise any extended release polymers.

Another one object of the present invention is to provide the compressible property of metformin.

As used herein, the term "metformin" includes metformin and pharmaceutically acceptable salts thereof. The pharmaceutically acceptable salts comprise hydrochloride, hydrobromide, fumarate, malonate, malate, succinate, lactate, glycolate, maleate, citrate, and aspartate. The pharmaceutically acceptable salts of metformin may be present in hydrous or anhydrous forms. They may also be present in crystalline or amorphous forms. In this invention, preferably metformin hydrochloride is used.

As used herein, the term "sitagliptin" includes sitagliptin and pharmaceutically acceptable salts thereof. The pharmaceutically acceptable salts comprise hydrochloride, hydrobromide, phosphate, sulphate, mesylate, besylate, tosylate, fumarate, malonate, malate, succinate, lactate, glycolate, maleate, citrate, and aspartate. The pharmaceutically acceptable salts of sitagliptin may be present in hydrous or anhydrous forms. The hydrate forms may be monohydrate or dihydrate forms. The pharmaceutically acceptable salts of sitagliptin may also be present in crystalline or amorphous forms. In this invention, preferably sitagliptin malate is used.

Sitagliptin and metformin combination has incompatibility and stability problems due to interactions between active agents. Separating metformin and sitagliptin in such a way that their interaction is eliminated is an effective solution so as to overcome these problems.

An osmotic tablet is one of the advanced inventions in controlled release oral drug delivery systems with the property of utilizing osmotic pressure as the driving force. The formulation of osmotic tablet mainly comprises of an osmotic core, which is coated with a semipermeable membrane, and passageway on the membrane coating. After orally taking, as soon as the tablet comes into contact with body fluids in stomach, body fluids pass through the semipermeable membrane and dissolves the active ingredient in the core. Then, an active agent can be released through one or more passageways in the membrane at a controlled rate due to the osmotic gradient generated across the semipermeable membrane coat.

Osmotic tablets also have advantages in preparing formulations. The release rate of osmotic tablet systems is highly predictable and can be programmed by modulating the release control parameters without extended release polymers. Also, for oral osmotic tablet systems, drug release is independent of gastric pH and hydrodynamic conditions.

The term "passageway" as used herein refers to and comprises any suitable means for releasing the contents of the core into the surrounding media. The term includes passages, apertures, bores, holes, openings and the like, created through the semipermeable membrane coating and forming a connection between the core and the surrounding media. The passageway can be of any shape. The passageway may be formed by mechanical drilling or laser drilling. Based on desired drug release profile, the number and diameter of the passageway may be adjusted. However, the diameter of the passageway should not be large enough to allow body fluids to enter the drug delivery system by the process of convection.

According to the present invention, there is provided an extended release osmotic tablet dosage form comprising metformin and sitagliptin as active agents and an osmotic agent; wherein said dosage form comprises
a) a core having metformin and the osmotic agent
b) a semipermeable membrane coating surrounding the core, and
c) an immediate release drug coating having sitagliptin surrounding the semipermeable membrane coating and wherein the amount of metformin is between 40.0% and 90.0% by weight of the dosage form, the amount of sitagliptin is between 2.0% and 15.0% by weight of the dosage form and wherein the amount of the osmotic agent in the core is between 1.0% and 10.0% by weight and wherein the osmotic agents comprise sodium chloride, magnesium sulfate, magnesium chloride, calcium chloride, lithium chloride, potassium chloride, sorbitol, xylitol, glucose, fructose or mixtures thereof.

In one of the embodiments, the semipermeable membrane coating does not comprise metformin or sitagliptin.

The term "core" as used herein refers to a compact mass having a definite geometric shape such as tablets, granules, pellets, capsules.

According to one embodiment of the present invention, the compartments are in direct contact with each other. In this invention, the compartments are in the form of core or coating. The first compartment is a core which comprises metformin. The second compartment is semipermeable membrane coating, the third compartment is the immediate release drug coating.

Metformin has been widely used for lowering blood glucose in patients. However, being a short acting drug, metformin requires twice-daily or three-times-a-day dosing. The side effects of metformin in this dose regime are often gastrointestinal in nature (anorexia, nausea, vomiting and occasionally diarrhea, etc.). These side effects may be partially avoided by either reducing the initial or maintenance dose or using an extended release form. Another advantage of the extended release form is the reduction in the frequency of administration. In other words, an extended release form of metformin may provide greater convenience and the desired profile.

When metformin and sitagliptin are taken at the same time, there might be inconveniences due to the differences in the half-life duration of active agents. Thus, the pharmaceutical combination comprises metformin and sitagliptin wherein the pharmaceutical composition has compartments comprising active agents with different release profiles.

According to the present invention, the amount of metformin is between 40.0% and 90.0% by weight of the dosage form. The amount of sitagliptin is between 2.0% and 15.0% by weight of the dosage form.

According to one embodiment of the present invention, preferably, the amount of metformin is between 55.0% and 75.0% by weight of the dosage form and the amount of sitagliptin is between 4.0% and 9.0% by weight of the dosage form.

It is a further object of the present invention to provide a dosage form, as described above, comprising delivery of a first active drug as an extended release formulation for metformin, wherein said extended release mechanism is not regulated by an extended release polymer, in combination with delivery of a second active drug by immediate release comprising sitagliptin.

According to the present invention, the tablet does not comprise any extended release polymers, in particular not the extended release polymers that are used in the core.

In one embodiment, the tablet is substantially free of extended release polymers.

Said extended release polymers are selected from the group comprising hydroxypropyl methyl cellulose, hydroxyethyl cellulose, hydroxypropyl cellulose, methyl cellulose, galactomannanes, guar gum, carob gum, gum arabicum, alginates, pectins, polyvinylacetate, acrylic acid polymers, polyethylene oxide, white wax, bees wax, carnauba wax, stearic acid, palmitic acid, lauric acid, cetyl alcohol, cetostearyl alcohol, stearyl alcohol, ethyl cellulose, acrylic acid polymers and copolymers (available commercially under Eudragit<^{®}>brand) or mixtures thereof.

According to the present invention, the core comprises metformin. The immediate release drug coating comprises sitagliptin.

According to one embodiment of the present invention, the ratio of the core to the immediate release drug coating is in the range of between 20:1 and 1:20, preferably between 10:1 and 1:10, more preferably between 8:1 and 1:8 by weight. This ratio provides the desired dissolution profile of active agents and the desired combination efficacy.

According to this embodiment of the invention, the core is between 70.0% and 96.0%, preferably between 80.0% and 90.0% by weight of the dosage form.

According to this embodiment of the invention, the immediate release drug coating is between 5.0% and 20.0%, preferably between 8.0% and 16.0% by weight of the dosage form.

According to this embodiment of the invention, the semipermeable membrane coating is between 0.5% and 10.0%, preferably between 2.0% and 8.0% by weight of the dosage form.

According to one embodiment of the present invention, it was not necessary to use a disintegrant because each active ingredient was combined with excipients suitable for their psychochemical properties and the desired dissolution rate in the compartment they are placed in. So, the formulation is free of disintegrant.

According to one embodiment of the present invention, the said disintegrant is croscarmellose or sodium starch glycolate or alginic acid or sodium alginate or calcium alginate or mannitol or povidone.

According to one embodiment of the present invention, the dosage form further comprises at least one pharmaceutically acceptable excipient which is selected from the group comprising binders, osmotic agents, flux enhancers, gelling agents, pore formers, semipermeable membrane polymers, plasticizers, lubricants, coating agents, coloring agents, solvents or mixtures thereof.

Suitable binders are selected from the group comprising polyvinylpyrrolidone, microcrystalline cellulose, lactose monohydrate, starch, dibasic calcium phosphate, tribasic calcium phosphate, trehalose, isomalt, sodium carbonate, sodium bicarbonate, calcium carbonate, carboxymethyl cellulose, polydextrose, polyethylene oxide, hydroxypropyl methyl cellulose, methyl cellulose, polyethylene glycol or mixtures thereof.

According to one embodiment of the present invention, the amount of binders is between 1.0% and 20.0%, preferably it is between 1.0% and 10.0%, more preferably it is between 3.0% and 7.0% by weight of the dosage form.

According to one embodiment of the present invention, the binders are present in the core or immediate release drug coating or both. Preferably, it is present in the core.

According to one embodiment of the present invention, preferably the binder is polyvinylpyrrolidone.

The flowability and compressibility of metformin are poor. In the present invention, granulating metformin with the help of polyvinylpyrrolidone as binder provides the desired flowability and compressibility of metformin.

The osmotic agents create osmotic pressure and fasten wetting of the core. The osmotic agents in the core draw body fluids into the core tablet and creating an osmotic gradient across the semipermeable membrane coating.

According to the present invention; osmotic agents comprise sodium chloride, magnesium sulfate, magnesium chloride, calcium chloride, lithium chloride, potassium chloride, sorbitol, xylitol, glucose, fructose, - or mixtures thereof. The amount of the osmotic agents in the core is between 1.0% and 10.0% by weight.

The amount of the osmotic agents in the core is preferably 1.0% and 5.0% by weight.

According to one embodiment of the present invention, the osmotic agent is sodium chloride and it is present in the core.

According to one embodiment of the present invention, the flux enhancer is present in semipermeable membrane coating. Flux enhancers are water soluble substances that aid in drawing water from the surrounding media and are thereby helpful in manipulating the semipermeable membrane's permeability.

Suitable flux enhancers are selected from the group comprising triacetin, hydroxymethyl cellulose, hydroxypropyl methylcellulose, polyethylene glycol, hydroxypropylcellulose, propylene glycol, polyvinylpyrrolidone or mixtures thereof. Preferably, the flux enhancer is triacetin.

The gelling agents in the core start swelling immediately after the contact with body fluids. As the more body fluids are drawn into the core with the help of osmotic agents, the gelling agents swell more and create a pressure inside the core. That pressure pushes the drug out through the passageway.

Suitable gelling agents are selected from the group comprising polyoxyethylene, polyacrylamide, hydroxypropyl cellulose, hydroxyethyl cellulose, methyl cellulose, hydroxypropylmethyl cellulose, modified starch, acrylic acid/ethyl acrylate copolymers, polymethacrylate copolymers, trihydroxystearin, aluminum magnesium hydroxy stearate, polyvinyl alcohol or mixtures thereof.

According to this embodiment of the invention, the gelling agents are between 4.0% and 20.0%, preferably between 9.0% and 17.0% by weight of the dosage form. Preferably, the gelling agent is polyoxyethylene.

According to another embodiment of the present invention, the pore former may be used. The more pores the coating will have, the speed of the drug release will consequently increase. Pore formers dissolved away without swelling and provided more sustained release.

Suitable pore formers are selected from the group comprising lactose monohydrate, sodium chloride, talc, silicon dioxide, lactose, sucralose, copovidone, maltodextrin, xylitol, poloxamers, polydextrose, or mixtures thereof.

According to one embodiment of the present invention, the amount of pore formers is between 0.1% and 5.0%, preferably it is between 0.5% and 3.0% by weight of the dosage form.

According to one embodiment of the present invention, preferably, the pore former is lactose monohydrate and it is present in the immediate release drug coat.

According to another embodiment of the present invention, the semipermeable membrane coating does not comprise an active pharmaceutical agent and that typically will rapidly disperse or dissolves in water. The coating comprises a polymer that provides a semipermeable membrane forming.

The semipermeable membrane coating may be one or more of semipermeable membrane polymers. Suitable semipermeable membrane polymers are selected from the group comprising cellulose acetate, cellulose triacetate, agar acetate, amylose acetate, cellulose acetate ethyl carbamate, cellulose acetate phthalate, cellulose acetate methyl carbamate, cellulose acetate succinate, cellulose acetate butyrate, polymeric epoxides, copolymers of alkylene oxides and alkyl glycidyl ethers, polyglycols, polylactic acid, methacrylic acid methyl ester or mixtures thereof. Preferably, the semipermeable membrane polymer is cellulose acetate.

According to one embodiment of the present invention, the amount of the semipermeable membrane polymers is between 2.0% and 6.0% by weight of the dosage form.

According to one embodiment of the present invention, the semipermeable membrane coating further comprises at least one coating agent.

Permeability of the semipermeable membrane coating can be increased by adding plasticizers, which increases the water diffusion coefficient. Suitable plasticizers are selected from the group comprising polyethylene glycol, propylene glycol, triethyl citrate, dibutyl sebacate, phthalates or mixtures thereof.

According to one embodiment of the present invention, the amounts of plasticizers is between 0.01% and 8.0%, preferably it is between 0.1% and 5.0% by weight of the dosage form. Preferably, the plasticizer is polyethylene glycol.

Suitable lubricants are selected from the group comprising magnesium stearate, hydrophobic silicon dioxide, talc, sodium stearyl fumarate, polyethylene glycol, sodium lauryl sulphate, magnesium lauryl sulphate, fumaric acid, glyceryl palmitostearate, hydrogenated natural oils, zinc stearate, calcium stearate, silica, stearic acid, paraffin or mixtures thereof.

According to one embodiment of the present invention, the amount of lubricants is between 0.1% and 3.0% by weight of the dosage form. Preferably, the lubricant is magnesium stearate.

According to one embodiment of the present invention, the lubricants are present in the core or immediate release drug coating or both. Preferably, it is present in the core.

Suitable coating agents are selected from the group comprising, hydroxypropyl cellulose, candelilla wax, cellulose acetate, polyvinyl alcohol (PVA), polyethylene glycol (PEG), talc, hydroxypropyl methylcellulose, polymethacrylates, triacetin, glycerol triacetin, lactose monohydrate, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), all kinds of Opadry^{®}, macrogol or mixtures thereof.

Preferably, the coating agents are hydroxypropyl cellulose or candelilla wax or mixtures thereof.

Suitable coloring agents are selected from the group comprising titanium dioxide, ferric oxide, Food, Drug & Cosmetic (FD&C) dyes (such as; FD&C blue, FD&C green, FD&C red, FD&C yellow, FD&C lakes), ponceau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof. Preferably, the coloring agent is titanium dioxide.

Metformin is known to be very poorly compressible active substance for tableting process. As it is highly crystalline and has poor compaction properties, it is difficult to form tablets by direct compression or dry granulation. Metformin further, is a high dose drug that is hard to compress directly into tablets. On the other hand, it is known that conventional methods of dry granulation fail to provide a satisfactory solution to poor compaction properties of metformin. It has been found that tablet of the desired stability and improved dissolution rate may be obtained when metformin and sitagliptin are formulated in a wet granulation process.

According to one embodiment of the present invention, sitagliptin and metformin combination are stable. The combination does not show incompatibilities, degradation problems, or extraction problems with certain excipients such as polyvinylpyrrolidone, polyoxyethylene, sodium chloride, magnesium stearate, cellulose acetate, triacetin, polyethylene glycol, hydroxypropyl cellulose, lactose monohydrate, titanium dioxide, acetone, pure water or mixtures thereof.

According to one embodiment of the present invention, the core comprises 40.0 to 90.0 by weight of metformin, 1.0 % to 10.0 % by weight of polyvinylpyrrolidone, 4.0 % to 20.0 % by weight of polyoxyethylene, 1.0 % to 10.0 % by weight of sodium chloride, 0.1 % to 3.0 % by weight of magnesium stearate of the dosage form.

According to one embodiment of the present invention, the immediate release drug coating comprises 2.0 % to 15.0 I % by weight of sitagliptin, 0.1 % to 5.0 I % by weight of polyethylene glycol, 0.1 % to 5.0 I % by weight of hydroxypropyl cellulose, 0.1 % to 5.0 I % by weight of lactose monohydrate, 0.01 % to 2.0 % by weight of titanium dioxide of the dosage form.

According to one embodiment of the present invention, the compartments may be coated or it may be compressed as a layer.

According to another embodiment of the present invention, there is provided a process for the preparation of the extended release osmotic tablets comprising metformin and sitagliptin, wherein the osmotic tablet is prepared by a process comprising the steps of:
a) preparing a core
b) applying a semipermeable membrane coating surrounding the core
c) applying an immediate release drug coating comprising sitagliptin surrounding the semipermeable membrane coating
d) optionally, applying a polishing coating surrounding the immediate release drug coating Metformin and polyvinylpyrrolidone in pure water are granulated, then polyoxyethylene, sodium chloride and finally magnesium stearate are added into the mixture. After, the core is formed with the mixture.

The semipermeable membrane coating is formed surrounding the core. Acetate cellulose is dissolved in acetone, and triacetin and polyethylene glycol are added into the mixture to prepare a solution which is then sprayed onto the core.

The immediate release drug coating is formed surrounding the semipermeable membrane coating. The solution comprising polyethylene glycol, hydroxypropyl cellulose, lactose monohydrate is prepared. After that, sitagliptin and titanium dioxide are added into the solution and the immediate release drug coating having an active agent is obtained.

Optionally, the polishing coating is formed with candelilla wax. After, the polishing coating is sprayed over the immediate release drug coating.

### Example 1

| | **(%) amount (w/w)** |
|---|---|
| **Core** | **80.0** - **90.0** |
| Metformin | 55.0 - 75.0 |
| Binders | 3.0 - 8.0 |
| Osmotic agents | 2.0 - 6.0 |
| **Semipermeable membrane coating** | **2.0 - 8.0** |
| Coating agents | 2.0 - 6.0 |
| **Immediate release drug coating** | **8.0 - 16.0** |
| Sitagliptin | 4.0 - 9.0 |
| **Polishing coating (optionally)** | **0.1 - 3.0** |
| Candelilla wax | 0.1 - 3.0 |
| **Total composition** | **100** |

### Example 2

| | **(%) amount (w/w)** |
|---|---|
| **Core** | **70.0 - 96.0** |
| Metformin | 40.0 - 90.0 |
| Binders | 1.0 - 10.0 |
| Gelling agents | 4.0 - 20.0 |
| Osmotic agents | 1.0 - 10.0 |
| Lubricants | 0.1 - 3.0 |
| Solvent | q.s. |
| **Semipermeable membrane coating** | **0.5 - 10.0** |
| Semipermeable membrane polymers | 2.0 - 6.0 |
| Flux enhancers | 0.01 - 3.0 |
| Plasticizers | 0.01 - 3.0 |
| solvent | q.s. |
| **Immediate release drug coating** | **5.0 - 20.0** |
| Sitagliptin | 2.0 - 15.0 |
| Coating agents | 0.1 - 5.0 |
| Pore formers | 0.1 - 5.0 |
| Plasticizers | 0.1 - 5.0 |
| Coloring agents | 0.01 - 2.0 |
| solvent | q.s. |
| **Polishing coating (optionally)** | **0.1 - 3.0** |
| Candelilla wax | 0.1 - 3.0 |
| **Total composition** | **100** |

### Example 3

| | **(%) amount (w/w)** |
|---|---|
| **Core** | **70.0 - 96.0** |
| Metformin | 40.0 - 90.0 |
| Polyvinylpyrrolidone | 1.0 - 10.0 |
| Polyoxyethylene | 4.0 - 20.0 |
| Sodium chloride | 1.0 - 10.0 |
| Magnesium stearate | 0.1 - 3.0 |
| Pure water | q.s. |
| **Semipermeable membrane coating** | **0.5 - 10.0** |
| Cellulose acetate | 2.0 - 6.0 |
| Triacetin | 0.01 - 3.0 |
| Polyethylene glycol | 0.01 - 3.0 |
| Acetone | q.s. |
| **Immediate release drug coating** | **5.0 - 20.0** |
| Sitagliptin | 2.0 - 15.0 |
| Polyethylene glycol | 0.1 - 5.0 |
| Hydroxypropyl cellulose | 0.1 - 5.0 |
| Lactose monohydrate | 0.1 - 5.0 |
| Titanium dioxide | 0.01 - 2.0 |
| Pure water | q.s. |
| **Polishing coating (optionally)** | **0.1 - 3.0** |
| Candelilla wax | 0.1 - 3.0 |
| **Total composition** | **100** |

| | |
|---|---|
| q.s.: quantity sufficient | |

A process for preparing the tablet in example 3 comprises the following steps:

### Core

a) dissolving polyvinylpyrrolidone in pure water to obtain binder solution
b) granulating the binder solution and metformin
c) drying the granules, then sieving
d) adding polyoxyethylene and sodium chloride into the mixture and mixing
e) then, adding magnesium stearate and mixing
f) compressing the mixture to form a core

### Semipermeable membrane coating

g) dissolving cellulose acetate in acetone
h) adding triacetin and polyethylene glycol into the mixture and mixing to obtain a coating solution
i) coating the core with the coating solution

### Immediate release drug coating

j) preparing solution comprising polyethylene glycol, hydroxypropyl cellulose, lactose monohydrate
k) adding sitagliptin and titanium dioxide and mixing
l) coating the semipermeable membrane coating with the mixture prepared at the step (k)

### Polishing coating

m) optionally, coating the immediate release drug coating with candelilla wax

Then, determined side or sides of the obtained tablets are drilling by laser at the determined dimensions.

## Claims

1. An extended release osmotic tablet dosage form comprising metformin and sitagliptin as active agents and an osmotic agent; wherein said dosage form comprises
a) a core having metformin and the osmotic agent
b) a semipermeable membrane coating surrounding the core, and
c) an immediate release drug coating having sitagliptin surrounding the semipermeable membrane coating
and wherein the amount of metformin is between 40.0% and 90.0% by weight of the dosage form, the amount of sitagliptin is between 2.0% and 15.0% by weight of the dosage form and wherein the amount of the osmotic agent in the core is between 1.0% and 10.0% by weight and wherein the osmotic agents comprise sodium chloride, magnesium sulfate, magnesium chloride, calcium chloride, lithium chloride, potassium chloride, sorbitol, xylitol, glucose, fructose or mixtures thereof.

2. The extended release osmotic tablet dosage form according to claim 1, wherein the dosage form comprising;
a) a core having metformin, binders and osmotic agents
b) a semipermeable membrane coating surrounding the core, and semipermeable membrane coating having semipermeable membrane polymers, flux enhancers, plasticizers
c) an immediate release drug coating having sitagliptin, pore formers, plasticizers surrounding the semipermeable membrane coating.

3. The extended release osmotic tablet dosage form according to claim 1 or 2, wherein the ratio of the core to the immediate release drug coating is in the range of between 20:1 and 1:20 by weight.

4. The extended release osmotic tablet dosage form according to any of the preceding claims, wherein the core is between 70.0% and 96.0% by weight of the dosage form.

5. The extended release osmotic tablet dosage form according to claim any of the preceding claims, wherein the immediate release drug coating is between 5.0% and 20.0% by weight of the dosage form.

6. The extended release osmotic tablet dosage form according to claim any of the preceding claims, wherein the semipermeable membrane coating is between 0.5% and 10.0% by weight of the dosage form.

7. The extended release osmotic tablet dosage form according to any preceding claims, further comprising at least one pharmaceutically acceptable excipient which is selected from the group comprising binders, flux enhancers, gelling agents, pore formers, semipermeable membrane polymers, plasticizers, lubricants, coating agents, coloring agents, solvents or mixtures thereof.

8. The extended release osmotic tablet dosage form according to claim 7, wherein the semipermeable membrane polymers are selected from the group comprising cellulose acetate, cellulose triacetate, agar acetate, amylose acetate, cellulose acetate ethyl carbamate, cellulose acetate phthalate, cellulose acetate methyl carbamate, cellulose acetate succinate, cellulose acetate butyrate, polymeric epoxides, copolymers of alkylene oxides and alkyl glycidyl ethers, polyglycols, polylactic acid, methacrylic acid methyl ester or mixtures thereof.

9. The extended release osmotic tablet dosage form according to any preceding claims, wherein the core comprising;
40.0 % to 90.0 % by weight of metformin
1.0 % to 10.0 % by weight of polyvinylpyrrolidone
4.0 % to 20.0 % by weight of polyoxyethylene
1.0 % to 10.0 % by weight of sodium chloride
0.1 % to 3.0 %
by weight of magnesium stearate of the dosage form.

10. The extended release osmotic tablet dosage form according to any preceding claims, wherein the immediate release drug coating comprising;
2.0 % to 15.0 % by weight of sitagliptin
0.1 % to 5.0 % by weight of polyethylene glycol
0.1 % to 5.0 % by weight of hydroxypropyl cellulose
0.1 % to 5.0 % by weight of lactose monohydrate
0.01 % to 2.0 %
by weight of titanium dioxide of the dosage form.

11. The extended release osmotic tablet dosage form according to any preceding claims, wherein the dosage form comprising;
40.0 % to 90.0 % by weight of metformin
1.0 % to 10.0 % by weight of polyvinylpyrrolidone
4.0 % to 20.0 % by weight of polyoxyethylene
1.0 % to 10.0 % by weight of sodium chloride
0.1 % to 3.0 % by weight of magnesium stearate
2.0 % to 15.0 % by weight of sitagliptin
0.1 % to 5.0 % by weight of polyethylene glycol
0.1 % to 5.0 % by weight of hydroxypropyl cellulose
0.1 % to 5.0 % by weight of lactose monohydrate
0.01 % to 2.0 %by weight of titanium dioxide of the dosage form.

12. The process for the preparation of the extended release osmotic tablet dosage form according to any preceding claims, wherein the process comprising the following steps:
a) preparing a core
b) applying a semipermeable membrane coating surrounding the core
c) applying an immediate release drug coating comprising sitagliptin surrounding the semipermeable membrane coating
d) optionally, applying a polishing coating surrounding the immediate release drug coating.

## Patentansprüche

1. Osmotische Tablette mit verzögerter Freisetzung, umfassend Metformin und Sitagliptin als Wirkstoffe sowie ein osmotisches Mittel; wobei die Darreichungsform folgendes umfasst
a) einen Kern mit Metformin und dem osmotischen Mittel
b) eine den Kern umgebende semipermeable Membranbeschichtung und
c) eine sofort freisetzende Arzneimittelbeschichtung enthaltend Sitagliptin, die die semipermeablen Membranbeschichtung umgibt
und wobei die Menge an Metformin zwischen 40,0 Gew.-% und 90,0 Gew.-% der Darreichungsform, die Menge an Sitagliptin zwischen 2,0 und 15,0 Gew.-% der Darreichungsform und wobei die Menge des osmotischen Mittels im Kern zwischen 1,0 und 10,0 Gew.-% liegt und wobei die osmotischen Mittel Natriumchlorid, Magnesiumsulfat, Magnesiumchlorid, Calciumchlorid, Lithiumchlorid, Kaliumchlorid, Sorbitol, Xylitol, Glukose, Fruktose oder Mischungen davon umfassen.

2. Osmotische Tablette mit verzögerter Freisetzung gemäß Anspruch 1, wobei die Darreichungsform umfasst:
a) einen Kern mit Metformin, Bindemitteln und osmotischen Mitteln
b) eine den Kern umgebende semipermeable Membranbeschichtung und eine semipermeable Membranbeschichtung mit semipermeablen Membranpolymeren, Flussverstärkern, Weichmachern
c) eine sofort freisetzende Arzneimittelbeschichtung mit Sitagliptin, Porenbildnern, Weichmachern.

3. Osmotische Tablette mit verzögerter Freisetzung gemäß Anspruch 1 oder 2, wobei
das Verhältnis von Kern zu sofort freisetzender Arzneimittelbeschichtung im Bereich zwischen 20:1 und 1:20 liegt.

4. Die osmotische Tablettenformulierung mit verzögerter Freisetzung gemäß einem der vorstehenden Ansprüchen, wobei der Kern zwischen 70,0 Gew.-% und 96,0 Gew.-% der Darreichungsform ausmacht.

5. Die osmotische Tablettenformulierung mit verzögerter Freisetzung gemäß einem der
vorstehenden Ansprüchen, wobei die sofort freisetzende Arzneimittelbeschichtung zwischen 5,0 % und 20,0 Gew.-% der Darreichungsform ausmacht.

6. Die osmotische Tablettenformulierung mit verzögerter Freisetzung gemäß einem der
vorstehenden Ansprüchen, wobei die semipermeable Membranbeschichtung zwischen 0,5 % und 10,0 Gew.-% der Darreichungsform ausmacht.

7. Die osmotische Tablette mit verzögerter Freisetzung gemäß einem der vorstehenden Ansprüche, die ferner mindestens einen pharmazeutisch akzeptablen Hilfsstoff umfasst, der
aus der Gruppe ausgewählt ist, die aus Bindemitteln, Flussverstärkern, Geliermitteln, Porenbildnern, semipermeablen Membranpolymeren, Weichmachern, Schmiermitteln, Beschichtungsmitteln, Farbstoffen, Lösungsmitteln oder Mischungen davon ausgewählt ist.

8. Die osmotische Tablettenformulierung mit verzögerter Freisetzung gemäß Anspruch 7, wobei die semipermeablen Membranpolymere ausgewählt sind aus der Gruppe umfassend
Celluloseacetat, Cellulosetriacetat, Agarasacetat, Amyloseacetat, Celluloseacetat-Ethylcarbamat, Celluloseacetat-Phthalat, Celluloseacetat-Methylcarbamat, Celluloseacetat-Succinat, Celluloseacetat-Butyrat, polymeren Epoxide, Copolymeren von Alkylenoxiden und Alkylglycidylethern, Polyglykolen, Polymilchsäure, Methacrylsäuremethylester oder Mischungen davon.

9. Osmotische Tablettenformulierung mit verzögerter Freisetzung gemäß einem der vorstehenden Ansprüche, wobei der Kern umfasst:
40,0 bis 90,0 Gew.-% Metformin
1,0 bis 10,0 Gew.-% Polyvinylpyrrolidon
4,0 bis 20,0 Gew.-% Polyoxyethylen
1,0 bis 10,0 Gew.-% Natriumchlorid
0,1 bis 3,0 Gew.-% Magnesiumstearat der Darreichungsform.

10. Die osmotische Tablette mit verzögerter Freisetzung gemäß einem der vorstehenden Ansprüche, wobei die sofort freisetzende Arzneimittelbeschichtung umfasst:
2,0 bis 15,0 Gew.-% Sitagliptin
0,1 bis 5,0 Gew.-% Polyethylenglykol
0,1 bis 5,0 Gew.-% Hydroxypropylcellulose
0,1 bis 5,0 Gew.-% Lactose-Monohydrat
0,01 bis 2,0 Gew.-% Titandioxid der Darreichungsform.

11. Die osmotische Tablette mit verzögerter Freisetzung gemäß einem der vorstehenden Ansprüche, wobei die Darreichungsform umfasst:
40,0 bis 90,0 Gew.-% Metformin
1,0 bis 10,0 Gew.-% Polyvinylpyrrolidon
4,0 bis 20,0 Gew.-% Polyoxyethylen
1,0 bis 10,0 Gew.-% Natriumchlorid
0,1 bis 3,0 Gew.-% Magnesiumstearat
2,0 bis 15,0 Gew.-% Sitagliptin
0,1 bis 5,0 Gew.-% Polyethylenglykol
0,1 bis 5,0 Gew.-% Hydroxypropylcellulose
0,1 bis 5,0 Gew.-% Lctose-Monohydrat
0,01 bis 2,0 Gew.-% Titandioxid der Darreichungsform.

12. Verfahren zur Herstellung der osmotischen Tablette mit verzögerter Freisetzung
gemäß einem der vorstehenden Ansprüche, wobei das Verfahren die folgenden Schritte umfasst Schritte umfasst:
a) Herstellen eines Kerns
b) Aufbringen einer den Kern umgebenden semipermeablen Membranbeschichtung
c) Aufbringen einer sofort freisetzenden Arzneimittelbeschichtung, die Sitagliptin umfasst und den Kern umgibt
d) optionales Aufbringen einer Polierbeschichtung, die die sofort freisetzende Arzneimittelbeschichtung umgibt.

## Revendications

1. - Forme posologique de comprimé osmotique à libération prolongée, comprenant de la metformine et de la sitagliptine en tant que principes actifs et un agent osmotique, ladite forme posologique comprenant :
a) un noyau ayant de la metformine et l'agent osmotique ;
b) un enrobage de membrane semi-perméable entourant le noyau, et
c) un enrobage de médicament à libération immédiate ayant de la sitagliptine entourant l'enrobage de membrane semi-perméable,
et la quantité de metformine étant entre 40,0 % et 90,0 % en poids de la forme posologique, la quantité de sitagliptine étant entre 2,0 % et 15,0 % en poids de la forme posologique, et la quantité de l'agent osmotique dans le noyau étant entre 1,0 % et 10,0 % en poids, et les agents osmotiques comprenant le chlorure de sodium, le sulfate de magnésium, le chlorure de magnésium, le chlorure de calcium, le chlorure de lithium, le chlorure de potassium, le sorbitol, le xylitol, le glucose, le fructose ou leurs mélanges.

2. - Forme posologique de comprimé osmotique à libération prolongée, selon la revendication 1, la forme posologique comprenant :
a) un noyau ayant de la metformine, des liants et des agents osmotiques
b) un enrobage de membrane semi-perméable entourant le noyau, et un enrobage de membrane semi-perméable ayant des polymères de membrane semi-perméable, des activateurs de flux, des plastifiants
c) un enrobage de médicament à libération immédiate ayant de la sitagliptine, des agents porogènes, des plastifiants entourant l'enrobage de membrane semi-perméable.

3. - Forme posologique de comprimé osmotique à libération prolongée, selon la revendication 1 ou 2, dans laquelle le rapport du noyau à l'enrobage de médicament à libération immédiate est dans la plage d'entre 20:1 et 1:20 en poids.

4. - Forme posologique de comprimé osmotique à libération prolongée, selon l'une quelconque des revendications précédentes, dans laquelle le noyau représente entre 70,0 % et 96,0 % en poids de la forme posologique.

5. - Forme posologique de comprimé osmotique à libération prolongée, selon l'une quelconque des revendications précédentes, dans laquelle l'enrobage de médicament à libération immédiate représente entre 5,0 % et 20,0 % en poids de la forme posologique.

6. - Forme posologique de comprimé osmotique à libération prolongée, selon l'une quelconque des revendications précédentes, dans lequel l'enrobage de membrane semi-perméable représente entre 0,5 % et 10,0 % en poids de la forme posologique.

7. - Forme posologique de comprimé osmotique à libération prolongée, selon l'une quelconque des revendications précédentes, comprenant en outre au moins un excipient pharmaceutiquement acceptable qui est choisi dans le groupe comprenant les liants, les activateurs de flux, les agents gélifiants, les agents porogènes, les polymères de membrane semi-perméable, les plastifiants, les lubrifiants, les agents d'enrobage, les agents colorants, les solvants ou leurs mélanges.

8. - Forme posologique de comprimé osmotique à libération prolongée, selon la revendication 7, dans laquelle les polymères de membrane semi-perméable sont choisis dans le groupe comprenant l'acétate de cellulose, le triacétate de cellulose, l'acétate d'agar, l'acétate d'amylose, l'acétate éthyl carbamate de cellulose, l'acétate phtalate de cellulose, l'acétate méthyl carbamate de cellulose, l'acétate succinate de cellulose, l'acétate butyrate de cellulose, les époxydes polymères, les copolymères d'oxydes d'alkylène et d'alkyl glycidyl éthers, les polyglycols, l'acide polylactique, l'ester méthylique de l'acide méthacrylique ou leurs mélanges.

9. - Forme posologique de comprimé osmotique à libération prolongée, selon l'une quelconque des revendications précédentes, dans laquelle le noyau comprend :
40,0 % à 90,0 % en poids de metformine
1,0 % à 10,0 % en poids de polyvinylpyrrolidone
4,0 % à 20,0 % en poids de polyoxyéthylène
1,0 % à 10,0 % en poids de chlorure de sodium
0,1 % à 3,0 % en poids de stéarate de magnésium de la forme posologique.

10. - Forme posologique de comprimé osmotique à libération prolongée selon l'une quelconque des revendications précédentes, dans laquelle l'enrobage de médicament à libération immédiate comprend :
2,0 % à 15,0 % en poids de sitagliptine
0,1 % à 5,0 % en poids de polyéthylène glycol
0,1 % à 5,0 % en poids d'hydroxypropyl cellulose
0,1 % à 5,0 % en poids de lactose monohydraté
0,01 % à 2,0 % en poids de dioxyde de titane de la forme posologique.

11. - Forme posologique de comprimé osmotique à libération prolongée, selon l'une quelconque des revendications précédentes, dans laquelle la forme posologique comprend :
40,0 % à 90,0 % en poids de metformine
1,0 % à 10,0 % en poids de polyvinylpyrrolidone
4,0 % à 20,0 % en poids de polyoxyéthylène
1,0 % à 10,0 % en poids de chlorure de sodium
0,1 % à 3,0 % en poids de stéarate de magnésium
2,0 % à 15,0 % en poids de sitagliptine
0,1 % à 5,0 % en poids de polyéthylène glycol
0,1 % à 5,0 % en poids d'hydroxypropyl cellulose
0,1 % à 5,0 % en poids de lactose monohydraté
0,01 % à 2,0 % en poids de dioxyde de titane de la forme posologique.

12. - Procédé de préparation de la forme posologique de comprimé osmotique à libération prolongée, selon l'une quelconque des revendications précédentes, dans lequel le procédé comprend les étapes suivantes :
a) préparation d'un noyau
b) application d'un enrobage de membrane semi-perméable autour du noyau
c) application d'un enrobage de médicament à libération immédiate comprenant de la sitagliptine entourant l'enrobage de membrane semi-perméable
d) facultativement, application d'un enrobage de polissage entourant l'enrobage de médicament à libération immédiate.
